# EUROPÄISCHE PATENTSCHRIFT

(11) **EP 1 701 644 B1**
(45) Veröffentlichungstag und Bekanntmachung des Hinweises auf die Patenterteilung: **11.11.2009**
(21) Anmeldenummer: 04804791.4
(22) Anmeldetag: 13.12.2004
(51) Int. Cl.: A47L 15/00, A47L 15/10

(54) **GESCHIRRSPÜLMASCHINE ZUR VERWENDUNG VON OZON**
DISHWASHER USING OZONE
LAVE-VAISSELLE POUVANT UTILISER DE L'OZONE

(30) Priorität: 23.12.2003 DE 10360906
(43) Veröffentlichungstag der Anmeldung: 20.09.2006
(73) Patentinhaber: BSH Bosch und Siemens Hausgeräte GmbH, 81739 München (DE)
(72) Erfinder: JERG, Helmut, 89537 Giengen (DE); HEILIGENMANN, Caroline, 86470 Thannhausen (DE)
(86) Internationale Anmeldenummer: PCT/EP2004/053430
(87) Internationale Veröffentlichungsnummer: WO 2005/063109

(56) Entgegenhaltungen:
- EP-A- 0 487 474
- EP-A- 0 808 894
- WO-A-03/096863
- DE-A1- 3 000 826
- DE-A1- 3 232 057
- US-B1- 6 363 951
- PATENT ABSTRACTS OF JAPAN Bd. 1999, Nr. 10, 31. August 1999 (1999-08-31) -& JP 11 137882 A (HITACHI LTD), 25. Mai 1999 (1999-05-25)

## Beschreibung

Die Erfindung betrifft eine Geschirrspülmaschine mit einem Spülbehälter, Vorrichtungen zum Aufbringen von Spülflotte auf das Spülgut im Spülbehälter und wenigstens einem Spülprogramm mit Teilprogrammschritten, z. B. "Vorspülen", "Reinigen", "Zwischenspülen" und "Klarspülen", wobei ein Gas mit Oxidationswirkung der Spülflotte bzw. dem Rohwasser und/oder in den Innenraum des Spülbehälters zur Verwendung für einen Teilprogrammschritte mit Reinigungswirkung, z. B. "Reinigen", zusetzbar ist, um das Gas zumindest zur Reinigung und zur Desinfektion einsetzen zu können.

Ozon ist ein starkes Oxidationsmittel. Es ist allgemein bekannt, dass Ozon Eigenschaften wie Desodorierung, Entkeimung und Oxidation von organischen Stoffen aufweist. Ozon als starkes Oxidationsmittel bleicht viele organischen Farbstoffe und vernichtet Bakterien. Es wird als Desinfektionsmittel in Brauereien und Kühlräumen eingesetzt sowie zur Reinigung von Trinkwasser eingesetzt, wobei Ozon die Geschmacks-, Geruchs- und Farbstoffe sowie Bakterien, Viren von unreinem Wasser durch Oxydation zerstört.

Aus der US 6 363 951 B1 ist ein Ozonnierungssystem zum Waschen und Reinigen von Gegenständen bekannt. Das System enthält einen Behälter, z. B. ein Spülbecken in einer Küche, eine Ozonnierungseinheit und eine Diffusionseinrichtung. Im Behälter werden vorzugsweise Lebensmittel oder Geschirr mit Hilfe von Ozon gereinigt. Aus der Umgebung wird Luft angesaugt und durch die Ozonnierungseinheit geleitet. Der in der Luft enthaltene Sauerstoff wird in Ozon umgewandelt und in das Spülbecken geleitet. Hierfür sind zwei Möglichkeiten vorgesehen. Entweder wird es über ein zylinderförmiges Diffusionsteil am Ende eines Schlauches in das Wasser im Spülbecken geleitet. Das zylinderförmige Diffusionsteil hat Öffnungen, durch die das Ozon in das Wasser gelangt. Es besteht aus ozonresistentem, porösem Material, wie Kunststoff. In einer zweiten Ausführungsform ist eine Diffusionsplatte am Boden des Behälters angeordnet. Die Diffusionsplatte ist aus ozonresistentem, porösem Material, wobei die Größe der Öffnungen derart dimensioniert sind, dass nur Ozon in das Wasser gelangt und nicht umgekehrt Wasser durch die Öffnungen. Nachteiligerweise ist das Ozonnierungssystem nur für manuelle Reinigungsgänge einsetzbar und das Ozon kann nur für Reinigungszwecke eingesetzt werden.

Aus der US 2003/0080068 A1 ist eine Vorrichtung und ein Verfahren zum Behandeln von Luft und Wasser in Haushaltgeräten, z. B. Kühlschränke, Waschmaschinen und Wäschetrockner und Geschirrspülmaschinen, zur Desinfektion bekannt. Zur Erzeugung von Ozon wird ultraviolette Strahlung genutzt. Die ultraviolette Strahlung wird in einen Behälter mit Luft oder Wasser gesendet. Der Behälter mit Wasser ist durchlässig für ultraviolette Strahlung und vorzugsweise röhrenförmig. Das desinfizierte Wasser wird im Haushaltgerät eingesetzt und die mit Ozon versetzte Luft wird im Haushaltgerät verwendet, um den Innenraum in Haushaltgeräten zu desinfizieren. In Kühlschränken beispielsweise zu einer Zeit, während dessen der Kühlschrank nicht benutzt wird. Nachteiligerweise kann das durch ultraviolette Strahlen erzeugte Ozon nur zur Desinfektion aufgrund der vorhandenen Vorrichtungen wirksam eingesetzt werden.

Aus der DE 32 32 057 A1 ist eine Reinigungsmaschine, wie Waschmaschine oder Geschirrspülmaschine, bekannt, mit einem Vorratsbehälter für die Spülflotte und einem den Reinigungsvorgang steuerndem Programmsteuergerät bekannt, wobei ein Ozongenerator Ozon erzeugt, um das Reinigungsgut weitgehend keimfrei zu machen. Bei der Geschirrspülmaschine mit einer Spülkammer als Vorratsbehälter für das Geschirr ist in der Spülkammer wenigstens ein Sprüharm für das Reinigungsmittel angeordnet. Mit Hilfe einer Umwälzpumpe und einer Umwälzleitung wird die Spülflotte umgewälzt und mit dem Sprüharm über Düsen auf das Reinigungsgut, z. B. Teller, verteilt. Ein Ozongenerator nimmt über eine Entlüftungsleitung aus der Spülkammer Luft auf, diese wird vorher von einem Lufttrockner getrocknet. Die im Ozongenerator erzeugt Luft mit Ozon wird über eine Ozonzuführleitung zu einer Verzweigung an der Umwälzleitung geleitet, um die Spülflotte in der Umwälzleitung mit Ozon anzureichern. Die Verzweigung ist z. B. als Venturidüse ausgebildet. Die Ozonzugabe erfolgt nur in abschließenden Spülvorgängen. Nachteilig ist hierbei, dass aufgrund einfachen Zugabe des Ozons ausschließlich in der Umwälzleitung nur während abschließender Spülvorgänge das Ozon nur zur Desinfektion eingesetzt werden kann.

Bei der Reinigungsvorrichtung der DE 30 00 826 A1 wird Reinigungswasser aus deren Reinigungswassersammelraum über eine Reinigungswasserabzugsleitung einer Ozonerzeugungseinrichtung zugeführt. Dieser ist eine Vorkammer zugeordnet, in der Emulgator und eine Bromwasserstoffsäure oder Bromidtösung dem Reinigungswasser zudosiert werden. In der Ozonerzeugungseinrichtung aufbereitetes Reinigungswasser wird über eine Ableitung einer Sprüheinrichtung in den Innenraum des Gerätegehäuses der Reinigungsvorrichtung eingebracht.

Die Aufgabe der vorliegenden Erfindung ist es daher, eine Geschirrspülmaschine und einer zugehöriges Verfahren bereitzustellen, welche es erlaubt, Gase mit oxidierender Wirkung, insbesondere Ozon, wenigstens zur Reinigung und zur Desinfektion effektiv einzusetzen.

Diese Aufgabe wird durch die erfindungsgemäße Geschirrspülmaschine gemäß Anspruch 1 und das zugehörige Verfahren gemäß Anspruch 9 gelöst. Vorteilhafte Weiterbildungen der Erfindung sind durch die Unteransprüche und einen nebengeordneten Anspruch gekennzeichnet.

Die erfindungsgemäße Geschirrspülmaschine verfügt über einen Spülbehälter, Vorrichtungen zum Aufbringen von Spülflotte auf das Spülgut im Spülbehälter und wenigstens einem Spülprogramm mit Teilprogrammschritten, z. B. "Vorspülen", "Reinigen", "Zwischenspülen" und "Klarspülen", wobei ein Gas mit Oxidationswirkung der Spülflotte bzw. dem Rohwasser und/oder in den Innenraum des Spülbehälters zur Verwendung für einen Teilprogrammschritte mit Reinigungswirkung, z. B. "Reinigen", zusetzbar ist, um das Gas zumindest zur Reinigung und zur Desinfektion einsetzen zu können. Das Gas mit Oxidationswirkung ist im Zusammenwirken mit Nebel im Spülbehälter auf das Spülgut aufbringbar. Dadurch kann das Gas mit Oxidationswirkung, welches in den Nebeltröpfchen gelöst ist, auch im Inneren der Anschmutzungen des Spülgutes wirken, weil der Durchmesser der Nebeltröpfchen kleiner ist als Durchmesser der Poren der Anschmutzungen. Dazu ist der Nebel von einer Vernebelungseinrichtung, z. B: einem Ultraschallvernebler oder einer Vernebelungsdüse, aus Spülflotte bzw. Rohwasser, erzeugbar.

In einer weiteren Ausführungsform ist der Spülflotte bzw. dem Rohwasser, welches der Vernebelungseinrichtung zuführbar ist, bereits Gas mit Oxidationswirkung zugesetzt. Dadurch wird von der Vernebelungseinrichtung unmittelbar vorteilhafterweise Nebel mit gelöstem Gas mit Oxidationswirkung erzeugt.

Vorteilhafterweise ist der Spülflotte bzw. dem Rohwasser, welches der Vernebelungseinrichtung zuführbar ist, kein Gas mit Oxidationswirkung zugesetzt und das Gas mit Oxidationswirkung dem Innenraum des Spülbehälters direkt zusetzbar. Dies ermöglicht die direkte Zusetzung von Gas mit Oxidationswirkung in den Innenraum des Spülbehälters, wobei die Lösung des Gases mit Oxidationswirkung in den Nebeltröpfchen erst im Innenraum des Spülbehälters erfolgt.

In einer weiteren Ausführungsform ist das Gas mit Oxidationswirkung mit einer porösen Membran in die Spülflotte, vorzugsweise am Boden des Spülbehälters, der Spülflotte zur Lösung und Reaktion zusetzbar. Die Verwendung einer porösen Membran erlaubt eine sehr feine Verteilung des Gases mit Oxidationswirkung in der Spülflotte, so dass die Lösbarkeit und das Reaktionsvermögen verbessert wird.

Zweckmäßigerweise ist das Gas mit Oxidationswirkung mit einer Wasserstrahlpumpe mit Diffusor zur feinen Verteilung des Gases in der Spülflotte zur Lösung und Reaktion zusetzbar. Die Verwendung eines Diffusors erlaubt eine sehr feine Verteilung des Gases mit Oxidationswirkung in der Spülflotte, so dass die Lösbarkeit und das Reaktionsvermögen verbessert wird.

Vorzugsweise ist die Wasserstrahlpumpe in der Rohwasserzuleitung oder in der Umwälzleitung zum Beaufschlagen der Vorrichtungen zum Aufbringen von Spülflotte auf das Spülgut angeordnet, wobei vorzugsweise an einer Abzweigung nur ein Teil des Rohwassers bzw. der Spülflotte durch die Wasserstrahlpumpe leitbar ist. Dadurch kann dem gesamten Rohwasser oder der Spülflotte beim Umwälzen Gas mit Oxidationswirkung zugesetzt werden.

Zweckmäßigerweise ist zur Desinfektion auch der Spülflotte bzw. dem Rohwasser im Flottenspeicher und/oder Wärmetauscher Gas mit Oxidationswirkung zugesetzt, um Keimwachstum im Flottenspeicher und/oder Wärmetauscher zu unterbinden. Dies ermöglicht den hygienisch unbedenklichen Einsatz von Flottenspeichern und Wärmetauschern insbesondere auch bei längeren Aufbewahrungszeiten.

In einer vorteilhaften Ausführungsform ist das Gas mit Oxidationswirkung Ozon, welches in einem Ozongenerator erzeugbar ist. Ozon ist das stärkste gasförmige Oxidationsmittel, so dass eine besonders große Reinigungs- und Desinfektionswirkung davon ausgeht und des Weiteren sehr einfach in einem Ozongenerator vor Ort in einer Geschirrspülmaschine erzeugbar.

In einem erfindungsgemäßen Verfahren zur Verwendung eines Gases mit Oxidationswirkung in einer Geschirrspülmaschine mit wenigstens einem Spülprogramm mit Teilprogrammschritten, z. B. "Vorspülen", "Reinigen", "Zwischenspülen" und "Klarspülen", wird ein Gas mit Oxidationswirkung der Spülflotte bzw. dem Rohwasser und/oder in den Innenraum des Spülbehälters zur Verwendung für einen Teilprogrammschritt mit Reinigungswirkung, z. B. "Reinigen", zugesetzt, um das Gas zumindest zur Reinigung und zur Desinfektion einsetzen zu können, wobei das Gas mit Oxidationswirkung im Zusammenwirken mit Nebel als mit Ozon angereicherter Nebel im Innenraum des Spülbehälters auf das Spülgut aufgebracht wird, und wobei der Nebel von einer Vernebelungseinrichtung, z. B. einem Ultraschallvernebler oder einer Vernebelungsdüse, aus Spülflotte bzw. Rohwasser, erzeugt wird.

Dabei wird das Gas mit Oxidationswirkung im Zusammenwirken mit Nebel im Spülbehälter auf das Spülgut aufgebracht. Dadurch kann das Gas mit Oxidationswirkung, welches in den Nebeltröpfchen gelöst ist, auch im Inneren der Anschmutzungen des Spülgutes wirken, weil der Durchmesser der Nebeltröpfchen kleiner ist als Durchmesser der Poren der Anschmutzungen. Dazu wird der Nebel von einer Vernebelungseinrichtung, z. B. einem Ultraschallvernebler oder einer Vernebelungsdüse, aus Spülflotte bzw. Rohwasser, erzeugt.

Vorteilhafterweise ist der Spülflotte bzw. dem Rohwasser, welches der Vernebelungseinrichtung zugeführt wird, bereits Gas mit Oxidationswirkung zugesetzt. Dadurch wird von der Vernebelungseinrichtung unmittelbar vorteilhafterweise Nebel mit gelöstem Gas mit Oxidationswirkung erzeugt.

In einer vorteilhaften Ausführungsform ist der Spülflotte bzw. dem Rohwasser, welches der Vernebelungseinrichtung zugeführt wird, kein Gas mit Oxidationswirkung zugesetzt und das Gas mit Oxidationswirkung wird dem Innenraum des Spülbehälters direkt zugesetzt. Dies ermöglicht die direkte Zusetzung von Gas mit Oxidationswirkung in den Innenraum des Spülbehälters, wobei die Lösung des Gases mit Oxidationswirkung in den Nebeltröpfchen erst im Spülbehälter erfolgt.

Zweckmäßigerweise wird das Gas mit Oxidationswirkung mit einer porösen Membran, vorzugsweise am Boden des Spülbehälters, der Spülflotte zur Lösung und Reaktion zugesetzt. Die Verwendung einer porösen Membran erlaubt eine sehr feine Verteilung des Gases mit Oxidationswirkung in der Spülflotte, so dass die Lösbarkeit und das Reaktionsvermögen verbessert werden.

Vorteilhafterweise wird das Gas mit Oxidationswirkung mit einer Wasserstrahlpumpe mit Diffusor zur feinen Verteilung des Gases in der Spülflotte zur Lösung und Reaktion zugesetzt. Die Verwendung eines Diffusors erlaubt eine sehr feine Verteilung des Gases mit Oxidationswirkung in der Spülflotte, so dass die Lösbarkeit und das Reaktionsvermögen verbessert wird.

Zweckmäßigerweise wird durch die Zugabe von Tensiden zur Spülflotte die Oberflächenspannung der Spülflotte abgesenkt und dadurch die Wirkung des Ultraschallvemeblers erhöht.

In einer vorteilhaften Ausführungsform ist das Gas mit Oxidationswirkung Ozon, welches in einem Ozongenerator erzeugt wird. Ozon ist das stärkste gasförmige Oxidationsmittel, so dass eine besonders große Reinigungs- und Desinfektionswirkung davon ausgeht und des Weiteren sehr einfach in einem Ozongenerator vor Ort in einer Geschirrspülmaschine erzeugbar.

In einer erfindungsgemäßen Verwendung von mit Ozon angereichertem Nebel ist in einem Spülbehälter einer Geschirrspülmaschine mit Ozon zumindest eine Reinigung des Spülgutes möglich.

Nachfolgend wird die Erfindung anhand eines Ausführungsbeispiels unter Bezugnahme auf Zeichnungen näher erläutert. Es zeigt:

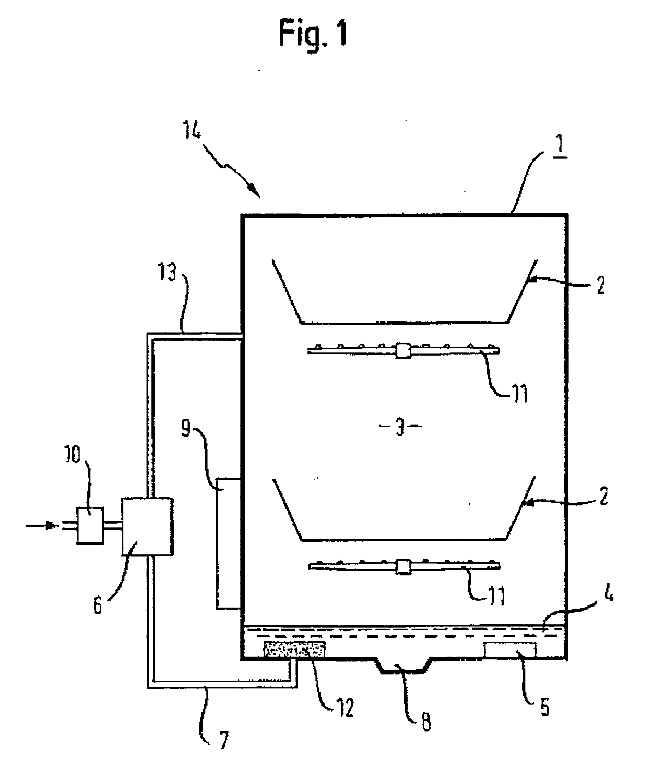

Fig. 1 einen Querschnitt durch einen Spülbehälter einer erfindungsgemäßen Geschirrspülmaschine mit Ozongenerator.

Gase mit Oxidationswirkung, z. B. Ozon und Chlor, entfalten vielfältige Wirkungen. Sie können zur Reinigung, Entfärbung (Bleichung), Desodorierung und Desinfektion eingesetzt werden. Ozon O, als aktiver Sauerstoff und instabile Modifikation des Sauerstoffs O₂ ist ein sehr effizientes Oxidationsmittel und übertrifft dabei Chlor um das 1,5-fache. Im Gegensatz zum Einsatz von Chlor entstehen keine umweltschädlichen Verbindungen. Ozon kann am Ort des Gebrauchs in der Geschirrspülmaschine mit einem Ozongenerator einfach und preiswert erzeugt werden. Als Ozongenerator kann z. B. eine Siemensröhre verwendet werden, welche über eine stille elektrische Entladung mit Hochspannung aus Sauerstoff in der Luft Ozon erzeugt. Ozon wird deshalb in Geschirrspülmaschinen als Gas mit Oxidationswirkung vorzugsweise eingesetzt.

Die zu entfernenden Anschmutzungen auf dem Spülgut bei Geschirrspülmaschinen sind organische Verbindungen, insbesondere Eiweiß- und Fettanschmutzungen, z. B. in Form von Milch, Margarine, Fleisch- oder Gemüseresten. Diese organischen Verbindungen werden durch das Oxidationsmittel Ozon teiloxidiert (primäre, direkte Reaktion des Ozons) und damit gereinigt. Beim Lösen von Ozon in Wasser bilden sich durch eine chemische Reaktion OH-Radikale (Hydroxyl-Radikale), welche mit organischen Verbindungen reagieren, d. h. es handelt sich um eine Reaktion sekundärer Oxidantien, die sich beim Ozonzerfall bilden (OH -Radikale). Diese Reaktion sekundärer Oxidantien wird als OH-Radikal-Reaktion bezeichnet. Ein Spezialfall der OH-Radikal-Reaktion ist die Ozonanlagerung an eine Doppelbindung als Ozonolyse.

Des Weiteren kann mit Ozon eine Entfärbung (Bleichung) von organischen Verfärbungen erreicht werden, die z. B. aus Schwarztee resultieren. Die organischen Verfärbungen werden oxidiert, wodurch keine zusätzlichen, umweltschädlichen und teuren Bleichmittel, z. B. Natriumperborat Monohydrat und Aktivator TAED, benötigt werden. Außerdem kann mit Ozon eine Desodorierung erreicht werden, so dass spülmaschinentypische Gerüche im Spülbehälter leicht entfernt werden können. Ozon hat auch eine desinfizierende Wirkung. Dadurch kann das Keimwachstum stark eingeschränkt oder ganz unterdrückt werden, was die hygienischen Verhältnisse stark verbessert. Vorteilhafterweise können dadurch bei neuen Trocknungsverfahren, die in einem Luftkreislauf feuchte Luft aus dem Spülbehälter entnehmen und trockenen und warme Luft wieder in den Spülbehälter einleiten, die Erwärmungstemperaturen in einen Teilprogrammschritt, z. B. "Reinigen" oder "Klarspülen", niedrig gehalten werden, weil eine bloße starke Erhitzung zur Desinfektion nicht mehr erforderlich ist. Dies ermöglicht eine erhebliche Energieeinsparung.

In einer erfindungsgemäßen Geschirrspülmaschine 14 mit Spülbehälter 1 mit Innenraum 3, Geschirrkorb 2, Pumpensumpf 8 und Sprüharmen 11, z. B. insbesondere als rotierende Sprüharme oder auch als nicht beweglicher Sprühboden, wird deshalb Ozon im Allgemeinen mit den übrigen Bestandteilen der Luft während eines Teilprogrammschrittes mit Reinigungswirkung, z. B. "Reinigen", der Spülflotte 4 und/oder dem Innenraum 3 des Spülbehälters 1 zugesetzt. Es haben auch die Teilprogrammschritte "Zwischenspülen" und "Klarspülen" Reinigungswirkung zur Entfernung von Restanschmutzungen sowie der Teilprogrammschritt "Vorspülen" zum Entfernen der gröbsten Verschmutzungen. Dadurch kann das Ozon sowohl insbesondere seine Reinigungs- und Desinfektionswirkung als auch seine Entfärbungs- und Desodorierungsfunktion nutzbar entfalten. Das Ozon und normalerweise die anderen Bestandteile der Luft wird hierzu entweder in die Spülflotte 4 zur Reaktion und/oder Lösung eingebracht oder in den Innenraum 3 des Spülbehälter 1 eingeleitet. Das Einbringen des Ozons in die Spülflotte 4 erfolgt z. B. mit einer porösen Membran 12 (Fritte oder Sprudelstein) am Boden des Spülbehälters 1. Hierzu wird das aus dem Sauerstoff der Luft mit dem Ozongenerator 6 gewonnene Ozon in die poröse Membran 12 über die Zuführleitung 7 eingeleitet. Hierzu wird z. B. ergänzend eine Luftpumpe 10 verwendet. Aufgrund der mikroskopisch kleinen Poren der Membran 12 gelangen sehr kleine Luftbläschen mit Ozon in die Spülflotte 4, was die Lösbarkeit und die Reaktionsfähigkeit aufgrund des größeren Verhältnisses aus Oberfläche zu Luftvolumen erhöht. Beim Einleiten von Luft mit Ozon in den Innenraum 3 des Spülbehälters 1 löst und reagiert das Ozon mit der Spülflotte 4 beim Betätigen der Sprüharme 4.

Außerdem kann mit einer Wasserstrahlpumpe die Luft mit Ozon in die Spülflotte 4 eingesaugt werden, wobei unter Spülflotte 4 in diesem Zusammenhang auch das Rohwasser, welches als Spülflotte 4 verwendet wird, verstanden wird. Vorteilhafterweise verfügt die Wasserstrahlpumpe nach der düsenförmigen Verengung mit Unterdruck zum Ansaugen der Luft über einen Abschnitt mit deutlich erhöhtem Querschnitt als Diffusor. Durch die feine Verteilung des Ozons in der Wasserstrahlpumpe im Diffusor wird die Lösbarkeit des Ozons im Wasser erhöht auch die Bildung von OH -Radikalen erleichtert. Die Wasserstrahlpumpe kann sowohl in der Rohwasserzuleitung für die Spülflotte 4 als auch in der Umwälzleitung der Umwälzpumpe zum Beaufschlagen der Sprüharme 11 enthalten sein (nicht dargestellt). Dabei wird vorzugsweise nicht die gesamte in der Umwälzleitung enthaltene Spülflotte durch die Wasserstrahlpumpe geleitet, sondern über eine Abzweigung ein Teil davon, wobei z. B. an der Abzweigung ein Steuerventil vorhanden ist, so dass der Anteil, welcher durch die Wasserstrahlpumpe geleitet wird, geregelt werden kann. Damit ist die in die Spülflotte eingebrachte Ozonmenge regelbar (nicht dargestellt).

Ein weiterer Vorteil der Ozonierung der Spülflotte 4 besteht darin, dass die Spülflotte 4 besser in einem Flottenspeicher (nicht dargestellt) zwischengespeichert werden kann. Ein Flottenspeicher dient dazu wenigstens einen Teil der Spülflotte 4, der nach der Ausführung eines Teilprogrammschrittes, z. B. "Klarspülen", nicht mehr benötigt wird und normalerweise mit der Laugenpumpe (nicht dargestellt) abgepumpt wird, zur Wiederverwendung in einem nachfolgenden Teilprogrammschritt, z. B. "Vorspülen", zwischengespeichert wird. Problematisch ist hierbei, dass im Flottenspeicher bei längeren Aufbewahrungszeiten ein starkes Wachstum von Bakterien und Pilze eintritt und dadurch die Wiederverwendung der Spülflotte im Flottenspeicher aus hygienischen Gründen problematisch oder ausgeschlossen ist. Die mit Ozon desinfizierte Spülflotte unterbindet ein starkes Wachstum von Bakterien und Pilzen im Flottenspeicher und ermöglicht so in vorteilhafter Weise die problemlose Speicherung und Wiederverwendung von Spülflotte in einem Flottenspeicher.

In einer ergänzenden Ausführungsform verfügt die erfindungsgemäße Geschirrspülmaschine 14 über einen Wärmetauscher 9 an einer Wandung des Spülbehälters 1. Der Wärmetauscher 9 wird mit kalten Rohwasser befüllt, damit an der Wandung des Spülbehälters 1 eine kalte Kondensationsfläche während des Teilprogrammschritts "Trocknen" entsteht, um die Trocknungsleistung zu erhöhen. Vorzugsweise wird auch das Rohwasser im Wärmetauscher 9 insbesondere vor dem Befüllen mit einer Wasserstrahlpumpe oder durch eine poröse Membran im Wärmetauscher 9 mit Ozon angereichert. Dadurch wird das Wachstum von Bakterien und Pilzen wegen der desinfizierenden Wirkung des Ozons im Wärmetauscher 9 vermieden, wodurch das Wasser des Wärmetauschers 9 problemlos als Spülflotte verwendet werden kann. Außerdem entfaltet in vorteilhafter Weise aufgrund der Ozonanreicherung dieses Wasser sämtliche Wirkungen des Ozons bei der Verwendung als Spülflotte 4.

In einer besonders vorteilhaften Ausführungsform der Erfindung kann mit einer Vernebelungseinrichtung, z. B. ein Ultraschallvernebler 5 oder einer Vernebelsungsdüse, Nebel, d. h. kleine Tröpfchen in der Luft, im Innenraum 3 des Spülbehälters 1 erzeugt werden. Hierzu ist vorzugsweise am Boden des Spülbehälters 1 z. B. ein Ultraschallvernebler 5 angeordnet. Der Ultraschallvernebler 5 vernebelt einen Teil der Spülflotte 4, wobei sich der Nebel im Spülbehälter 1 verteilt. Das Einbringen von Ozon in die Spülflotte 4 wird entweder dadurch ausgeführt, dass die Spülflotte 4 im Spülbehälter 1- wie oben beschrieben - bereits mit Ozon und daraus resultierend auch mit Hydroxyl-Radikalen angereichert ist oder es wird mit dem Ozongenerator 6 über die Zufuhrleitung 13 ozonhaltige Luft direkt in den Spülbehälter 1 eingeleitet. Der Ozongenerator entnimmt vorzugsweise die Luft aus dem Spülbehälter 1, damit im Spülbehälter 1 kein Überdruck entsteht (nicht dargestellt). Im letztgenannten Fall lagert sich das Ozon an die feinen Tröpfchen an, löst sich in den Tröpfchen und es bilden sich Hydroxyl-Radikale. Zur besseren Verteilung des Nebels im Spülbehälter 1 wird die Umwälzpumpe (nicht dargestellt) aktiviert und die Sprüharme 11 bewegen sich dadurch, was eine Luftströmung und Wirbelbildung im Spülbehälter 1 zur besseren Verteilung bewirkt.

In einer ergänzenden vorteilhafte Ausgestaltung können die Sprüharme 11 motorisch, z. B. mit einem Elektromotor, bewegt werden, ohne dass die Umwälzpumpe eingeschaltet wird und im Spülbehälter 1 die Spülflotte 4 über Düsen an den Sprüharme 11 verteilt wird. Damit kann eine bessere Verteilung des Nebels im Innenraum 3 des Spülbehälters 1 mit den Sprüharmen 11 erreicht werden, ohne dass ein Teil des Nebels von der über die Sprüharme 11 verteilten Spülflotte 4 wieder aufgenommen wird. Hierbei können die Sprüharme 11 dahingehend konstruktiv gestaltet sein, dass diese eine optimierte Ventilatorwirkung entfalten.

Die Verschmutzungen am Spülgut (nicht dargestellt), z. B. angetrocknete Verschmutzungen, haben Poren mit einem Durchmesser von im Allgemeinen ungefähr 6 µ m. Die Tröpfchen des Nebels im Spülbehälter 1 haben einen Durchmesser von im Allgemeines 3 µm. Dadurch können die Nebeltröpfchen in die feinen Poren der Verschmutzungen des Spülgutes eindringen und zusätzlich neben der Wirkung an der Oberfläche in besonders vorteilhafter und effektiver Weise auch im Inneren der Verschmutzungen wirken. Dies ermöglicht eine Teiloxidation der Verschmutzungen im Inneren durch das Ozon in den Nebeltropfen und eine Reaktion der Hydroxyl-Radikale mit der Verschmutzung, z. B. als Ozonolyse. Dadurch kann die Reinigungsleistung mit Ozon wesentlich verbessert werden, insbesondere auch bei angetrockneten Verschmutzungen, der bisher nur sehr schwer zu entfernen war.

Die Ozonkonzentration in der Spülflotte klingt durch Ausgasen, Reaktion mit organischen Verbindungen oder dem Zerfall des Ozons zu sekundären Oxidantien ab. Die Halbwertszeit von mit Ozon begasten Wasser liegt zwischen einer und zwanzig Minuten. Die Reaktionen des Ozons in der Spülflotte sind vom pH-Wert abhängig. In saurem Milieu ist das Ozon stabiler. Deshalb kann durch Zugabe von z. B. Klarspülern, die z. B. Zitronensäure enthalten, die Halbwertszeit von Ozon erhöht werden und damit insbesondere seine Reinigungs- und Desinfektionswirkung als auch seine Entfärbungs- und Desodorierungsfunktion verbessert werden. Deshalb wird vorzugsweise Klarspülmittel, z. B. im Teilprogrammschritt □Klarspülen□, während der Ozonierung zugegeben, um die Wirkungen des Ozons zu verbessern (nicht dargestellt).

Die Zugabe von Tensiden, welche in Klarspülern und Reinigungsmitteln enthalten sind, senken die Oberflächenspannung der Spülflotte und verbessern damit die Winkung des Ultraschallverneblers. Die größere Menge an Nebel im Spülbehälter 1 erhöht damit die Wirkungen des Ozons. Aus diesem Grund werden vorzugsweise Tenside während der Ozonierung mit Nebel zugegeben (nicht dargestellt).

Die erfindungsgemäße Geschirrspülmaschine ermöglicht die Ausnutzung sämtlicher Wirkungen von Ozon, insbesondere seine Reinigungs- und Desinfektionswirkung als auch seine Entfärbungs- und Desodorierungsfunktion, weil das Ozon in Teilprogrammschritten zugesetzt wird, die sämtliche Wirkungen des Ozons ausnützen können. In besonders vorteilhafter Weise wirkt das Ozon über Nebeltröpfchen auf die Verschmutzung des Geschirrs ein, so dass die Wirkungen des Ozons nicht nur auf der Oberfläche der Verschmutzung, sondern über Poren auch im Inneren der Verschmutzung eintreten, weil der Durchmesser der Nebeltröpfchen kleiner ist als der Durchmesser der Poren der Verschmutzung. Insbesondere bei angetrockneten Anschmutzungen, die bisher nur sehr schwer zu entfernen waren, ist eine erhebliche Steigerung des Reinigungsgrades erreichbar.

## Patentansprüche

1. Geschirrspülmaschine (14) mit einem Spülbehälter (1), Vorrichtungen (11) zum Aufbringen von Spülflotte (4) auf das Spülgut im Spülbehälter (1) und wenigstens einem Spülprogramm mit Teilprogrammschritten, z. B. "Vorspülen", "Reinigen", "Zwischenspülen" und "Klarspülen", wobei ein Gas mit Oxidationswirkung der Spülflotte (4) bzw. dem Rohwasser und/oder in den Innenraum (3) des Spülbehälters (1) zur Verwendung für einen Teilprogrammschritt mit Reinigungswirkung, z. B. "Reinigen", zusetzbar ist, um das Gas zumindest zur Reinigung und zur Desinfektion einsetzen zu können, **dadurch gekennzeichnet, dass** das Gas mit Oxidationswirkung im Zusammenwirken mit Nebel im Innenraum (3) des Spülbehälters (1) als mit Ozon angereicherter Nebel auf das Spülgut aufbringbar ist, wobei der Nebel von einer Vernebelungseinrichtung, z. B. einem Ultraschallvernebler (5) oder einer Vemebelungsdüse, aus Spülflotte (4) bzw. Rohwasser, erzeugbar ist.

2. Geschirrspülmaschine (14) nach Anspruch 1, **dadurch gekennzeichnet, dass** der Spülflotte (4) bzw. dem Rohwasser, welches der Vernebelungseinrichtung (5) zuführbar ist, bereits Gas mit Oxidationswirkung zugesetzt ist.

3. Geschirrspülmaschine (14) nach Anspruch 1, **dadurch gekennzeichnet, dass** der Spülflotte (4) bzw. dem Rohwasser, welches der Vernebelungseinrichtung zuführbar ist, kein Gas mit Oxidationswirkung zugesetzt ist und das Gas mit Oxidationswirkung dem Innenraum (3) des Spülbehälters (4) direkt zusetzbar ist.

4. Geschirrspülmaschine (14) nach einem der vorhergehenden Ansprüche, **dadurch gekennzeichnet, dass** das Gas mit Oxidationswirkung mit einer porösen Membran (12) in die Spülflotte (4), vorzugsweise am Boden des Spülbehälters (1), der Spülflotte (4) zur Lösung und Reaktion zusetzbar ist.

5. Geschirrspülmaschine (14) nach einem der vorhergehenden Ansprüche, **dadurch** gekenntzeichnet, dass das Gas mit Oxidationswirkung mit einer Wasserstrahlpumpe mit Diffusor zur feinen Verteilung des Gases in der Spülflotte (4) zur Lösung und Reaktion zusetzbar ist.

6. Geschirrspülmaschine (14) nach einem der vorhergehenden Ansprüche, **dadurch gekennzeichnet, dass** die Wasserstrahlpumpe in der Rohwasserzuleitung oder in der Umwälzleitung zum Beaufschlagen der Vorrichtungen (11) zum Aufbringen von Spülflotte (4) auf das Spülgut angeordnet ist, wobei vorzugsweise an einer Abzweigung nur ein Teil des Rohwassers bzw. der Spülflotte (4) durch die Wasserstrahlpumpe leitbar ist.

7. Geschirrspülmaschine (14) nach einem der vorhergehenden Ansprüche, **dadurch gekennzeichnet, dass** zur Desinfektion auch der Spülflotte (4) bzw. dem Rohwasser im Flottenspeicher und/oder Wärmetauscher (9) Gas mit Oxidationswirkung zugesetzt ist, um Keimwachstum im Flottenspeicher und/oder Wärmetauscher (9) zu unterbinden.

8. Geschirrspülmaschine (14) nach einem der vorhergehenden Ansprüche, **dadurch gekennzeichnet, dass** das Gas mit Oxidationswirkung Ozon ist, welches in einem Ozongenerator (6) erzeugbar ist.

9. Verfahren zur Verwendung eines Gases mit Oxidationswirkung in einer Geschirrspülmaschine mit wenigstens einem Spülprogramm mit Teilprogrammschritten, z. B. "Vorspülen", "Reinigen", "Zwischenspülen" und "Klarspülen", wobei ein Gas mit Oxidationswirkung der Spülflotte (4) bzw. dem Rohwasser und/oder in den Innenraum (3) des Spülbehälters (1) zur Verwendung für einen Teilprogrammschritt mit Reinigungswirkung, z. B. "Reinigen", zugesetzt wird, um das Gas zumindest zur Reinigung und zur Desinfektion einsetzen zu können, **dadurch gekennzeichnet, dass** das Gas mit Oxidationswirkung im Zusammenwirken mit Nebel als mit Ozon angereicherter Nebel im Innenraum (3) des Spülbehälters (1) auf das Spülgut aufgebracht wird, wobei der Nebel von einer Vernebelungseinrichtung, z. B. einem Ultraschallvernebler (5) oder einer Vernebelungsdüse, aus Spülflotte (4) bzw. Rohwasser, erzeugt wird.

10. Verfahren nach Anspruch 9, **dadurch gekennzeichnet, dass** der Spülflotte (4) bzw. dem Rohwasser, welches der Vernebelungseinrichtung zugeführt wird, bereits Gas mit Oxidationswirkung zugesetzt ist.

11. Verfahren nach Anspruch 9, **dadurch gekennzeichnet, dass** der Spülflotte (4) bzw. dem Rohwasser, welches der Vernebelungseinrichtung zugeführt wird, kein Gas mit Oxidationswirkung zugesetzt ist und das Gas mit Oxidationswirkung dem Spülbehälter (1) direkt zugesetzt wird.

12. Verfahren nach einem der Ansprüche 9 bis 11, **dadurch gekennzeichnet, dass** das Gas mit Oxidationswirkung mit einer porösen Membran (12), vorzugsweise am Boden des Spülbehälters (1), der Spülflotte (4) zur Lösung und Reaktion zugesetzt wird.

13. Verfahren nach einem der Ansprüche 9 bis 12, **dadurch gekennzeichnet, dass** das Gas mit Oxidationswirkung mit einer Wasserstrahlpumpe mit Diffusor zur feinen Verteilung des Gases in der Spülflotte (4) zur Lösung und Reaktion zugesetzt wird.

14. Verfahren nach einem der Ansprüche 9 bis 13, **dadurch gekennzeichnet, dass** durch die Zugabe von Tensiden zur Spülflotte (4) die Oberflächenspannung der Spülflotte (4) abgesenkt wird und **dadurch** die Wirkung des Ultraschallverneblers (5) erhöht wird.

15. Verfahren nach einem der Ansprüche 9 bis 14, **dadurch gekennzeichnet, dass** das Gas mit Oxidationswirkung Ozon ist, welches in einem Ozongenerator (6) erzeugt wird.

## Claims

1. Dishwashing machine (14) with a rinsing container (1), devices (11) for application of rinsing solution (4) to the rinsing stock in the rinsing container (1) and at least one rinsing program with part program steps, for example "pre-rinsing", "cleaning", "intermediate rinsing" and "clear rinsing", wherein a gas with oxidation action can be added to the rinsing solution (4) or to the untreated water and/or to the interior space (3) of the rinsing container (1) for use for a part program step with cleaning action, for example "cleaning", in order to be able to use the gas at least for cleaning and for disinfecting, **characterised in that** the gas with oxidation action can be applied to the rinsing stock in co-operation with mist, as mist enriched with ozone, in the interior space (3) of the rinsing container (1), wherein the mist can be produced by an atomising device, for example an ultrasonic atomiser (5) or an atomising nozzle, from rinsing solution (4) or raw water.

2. Dishwashing machine (14) according to claim 1, **characterised in that** gas with oxidation action is already added to the rinsing solution (4) or to the raw water, which can be supplied to the atomising device (5).

3. Dishwashing machine (14) according to claim 1, **characterised in that** no gas with oxidation action is added to the rinsing solution (4) or to the untreated water, which can be supplied to the atomising device, and that the gas with oxidation action can be directly added to the interior space (3) of the rinsing container (4).

4. Dishwashing machine (14) according to any one of the preceding claims, **characterised in that** the gas with oxidation action can be added to the rinsing solution (4), by a porous membrane (12) in the rinsing solution (4), preferably at the base of the rinsing container (1), for dissolving and reaction.

5. Dishwashing machine (14) according to any one of the preceding claims, **characterised in that** the gas with oxidation action can be added by a water jet pump with diffuser for fine distribution of the gas in the rinsing solution (4), for dissolving and reaction.

6. Dishwashing machine (14) according to any one of the preceding claims, **characterised in that** the water jet pump is arranged in the raw water feed or in the circulating duct for loading of the devices (11) for applying rinsing solution (4) to the rinsing stock, wherein preferably only a part of the raw water or the rinsing solution (4) can be conducted by the water jet pump at a branch.

7. Dishwashing machine (14) according to any one of the preceding claims, **characterised in that**, for disinfecting, gas with oxidation action is also added to the rinsing solution (4) or the raw water in the solution reservoir and/or heat exchanger (9) in order to suppress bacterial growth in the solution reservoir and/or heat exchanger (9).

8. Dishwashing machine (14) according to any one of the preceding claims, **characterised in that** the gas with oxidation action is ozone which can be generated in an ozone generator (6).

9. Method of using a gas with oxidation action in a dishwashing machine with at least one rinsing program with part program steps, for example "pre-rinsing", "cleaning", "intermediate rinsing" and "clear rinsing", wherein a gas with oxidation action is added to the rinsing solution (4) or to the raw water and/or to the interior space (3) of the rinsing container (1) for use for a part program step with cleaning action, for example "cleaning", in order to be able to use the gas at least for cleaning and for disinfecting, **characterised in that** the gas with oxidation action is applied to the rinsing stock in co-operation with mist, as mist enriched with ozone, in the interior space (3) of the rinsing container (1), wherein the mist is produced by an atomising device, for example an ultrasonic atomiser (5) or an atomising nozzle, from rinsing solution (4) or raw water.

10. Method according to claim 9, **characterised in that** gas with oxidation action is already added to the rinsing solution (4) or to the raw water, which is supplied to the atomising device.

11. Method according to claim 9, **characterised in that** no gas with oxidation action is added to the rinsing solution (4) or to the raw water, which is supplied to the atomising device, and the gas with oxidation action is directly added to the rinsing container (1).

12. Method according to any one of claims 9 to 11, **characterised in that** the gas with oxidation action is added by a porous membrane (12), preferably at the base of the rinsing container (1), to the rinsing solution (4), for dissolving and reaction.

13. Method according to any one of claims 9 to 12, **characterised in that** the gas with oxidation action is added by a water jet pump with diffuser for fine distribution of the gas in the rinsing solution (4), for dissolving and reaction.

14. Method according to any one of claims 9 to 13, **characterised in that** the surface tension of the rinsing solution (4) is reduced by the addition of surfactants to the rinsing solution (4) and the action of the ultrasonic atomiser (5) is thereby increased.

15. Method according to any one of claims 9 to 14, **characterised in that** the gas with oxidation action is ozone which is generated in an ozone generator (6).

## Revendications

1. Lave-vaisselle (14) comprenant un réservoir de lavage (1), des dispositifs (11) pour l'application d'eau de lavage (4) sur l'article à laver dans le réservoir de lavage (1) et au moins un programme de lavage avec des étapes de programme partiel, par exemple "prélavage", "nettoyage", "lavage intermédiaire" et "rinçage", un gaz avec effet d'oxydation pouvant être ajouté à l'eau de lavage (4) ou à l'eau brute et/ou dans l'espace intérieur (3) du réservoir de lavage (1) pour utilisation pour une étape de programme partiel avec effet de nettoyage, par exemple "nettoyage", afin de pouvoir utiliser le gaz au moins pour le nettoyage et pour la désinfection, **caractérisé en ce que** le gaz à effet d'oxydation peut être appliqué sur l'article à laver conjointement avec du brouillard dans l'espace intérieur (3) du réservoir de lavage (1) sous forme de brouillard enrichi avec de l'ozone, le brouillard pouvant être généré par un dispositif de nébulisation, par exemple un nébulisateur à ultrasons (5) ou une buse de nébulisation, à partir d'eau de lavage (4) ou d'eau brute.

2. Lave-vaisselle (14) selon la revendication 1, **caractérisé en ce que** du gaz à effet d'oxydation est déjà ajouté à l'eau de lavage (4) ou à l'eau brute qui peut être amenée au dispositif de nébulisation (5).

3. Lave-vaisselle (14) selon la revendication 1, **caractérisé en ce que** aucun gaz à effet d'oxydation n'est ajouté à l'eau de lavage (4) ou à l'eau brute qui peut être amenée au dispositif de nébulisation et le gaz à effet d'oxydation peut être amené directement à l'espace intérieur (3) du réservoir de lavage (4).

4. Lave-vaisselle (14) selon l'une quelconque des revendications précédentes, **caractérisé en ce que** le gaz à effet d'oxydation peut être ajouté avec une membrane poreuse (12) dans l'eau de lavage (4), de préférence sur le fond du réservoir de lavage (1), à l'eau de lavage (4) pour la dissolution et la réaction.

5. Lave-vaisselle (14) selon l'une quelconque des revendications précédentes, **caractérisé en ce que** le gaz à effet d'oxydation peut être ajouté avec une pompe à jet d'eau avec diffuseur pour la fine répartition du gaz dans l'eau de lavage (4) pour la dissolution et la réaction.

6. Lave-vaisselle (14) selon l'une quelconque des revendications précédentes, **caractérisé en ce que** la pompe à jet d'eau est disposée dans la conduite d'arrivée d'eau brute ou dans la conduite de circulation pour l'alimentation des dispositifs (11) pour l'application d'eau de lavage (4) sur l'article à laver, seule une partie de l'eau brute ou de l'eau de lavage (4) pouvant être acheminée par la pompe à jet d'eau de préférence sur une bifurcation.

7. Lave-vaisselle (14) selon l'une quelconque des revendications précédentes, **caractérisé en ce que** du gaz à effet d'oxydation est ajouté pour la désinfection également à l'eau de lavage (4) ou à l'eau brute dans le réservoir d'eau et/ou l'échangeur de chaleur (9), afin d'empêcher la croissance de germes dans le réservoir d'eau et/ou l'échangeur de chaleur (9).

8. Lave-vaisselle (14) selon l'une quelconque des revendications précédentes, **caractérisé en ce que** le gaz à effet d'oxydation est de l'ozone qui peut être généré dans un générateur d'ozone (6).

9. Procédé pour utiliser un gaz à effet d'oxydation dans un lave-vaisselle comprenant au moins un programme de lavage avec des étapes de programme partiel, par exemple "prélavage", "nettoyage", "lavage intermédiaire" et "rinçage", un gaz à effet d'oxydation étant ajouté à l'eau de lavage (4) ou à l'eau brute et/ou dans l'espace intérieur (3) du réservoir de lavage (1) pour utilisation pour une étape de programme partiel avec effet de nettoyage, par exemple "nettoyage", afin de pouvoir utiliser le gaz au moins pour le nettoyage et pour la désinfection, **caractérisé en ce que** le gaz à effet d'oxydation est appliqué sur l'article à laver conjointement avec du brouillard sous forme de brouillard enrichi avec de l'ozone dans l'espace intérieur (3) du réservoir de lavage (1), le brouillard étant généré par un dispositif de nébulisation, par exemple un nébulisateur à ultrasons (5) ou une buse de nébulisation, à partir d'eau de lavage (4) ou d'eau brute.

10. Procédé selon la revendication 9, **caractérisé en ce que** du gaz à effet d'oxydation est déjà ajouté à l'eau de lavage (4) ou à l'eau brute qui est amenée au dispositif de nébulisation.

11. Procédé selon la revendication 9, **caractérisé en ce que** aucun gaz à effet d'oxydation n'est ajouté à l'eau de lavage (4) ou à l'eau brute qui est amenée au dispositif de nébulisation et le gaz à effet d'oxydation est ajouté directement au réservoir de lavage (1).

12. Procédé selon l'une quelconque des revendications 9 à 11, **caractérisé en ce que** le gaz à effet d'oxydation est ajouté avec une membrane (12) poreuse, de préférence sur le fond du réservoir de lavage (1), à l'eau de lavage (4) pour la dissolution et la réaction.

13. Procédé selon l'une quelconque des revendications 9 à 12, **caractérisé en ce que** le gaz à effet d'oxydation est ajouté avec une pompe à jet d'eau avec diffuseur pour la fine répartition du gaz dans l'eau de lavage (4) pour la dissolution et la réaction.

14. Procédé selon l'une quelconque des revendications 9 à 13, **caractérisé en ce que** la tension de surface de l'eau de lavage (4) est abaissée par l'addition d'agents tensio-actifs à l'eau de lavage (4) et l'effet du nébulisateur à ultrasons (5) est ainsi amélioré.

15. Procédé selon l'une quelconque des revendications 9 à 14, **caractérisé en ce que** le gaz à effet d'oxydation est de l'ozone, qui est généré dans un générateur d'ozone (6).
